# EUROPEAN PATENT SPECIFICATION

(11) **EP 3 090 744 B1**
(45) Date of publication and mention of the grant of the patent: **24.11.2021**
(21) Application number: 14876904.5
(22) Date of filing: 23.12.2014
(51) Int. Cl.: A61P 9/12, A61K 31/505, A61K 31/4184, A61K 9/20

(54) **PHARMACEUTICAL COMPLEX FORMULATION COMPRISING ANGIOTENSIN II RECEPTOR BLOCKER AND HMG-COA REDUCTASE INHIBITOR**
PHARMAZEUTISCHE KOMPLEXE FORMULIERUNG MIT ANGIOTENSIN-II-REZEPTOR-BLOCKER UND HMG-COA-REDUKTASE-HEMMER
FORMULATION COMPLEXE PHARMACEUTIQUE COMPRENANT UN INHIBITEUR DE RÉCEPTEUR D'ANGIOTENSINE II ET UN INHIBITEUR DE RÉDUCTASE HMG-COA

(30) Priority: 30.12.2013 KR 20130167406
(43) Date of publication of application: 09.11.2016
(73) Proprietor: Alvogen Korea Co., Ltd., Seoul 07326 (KR)
(72) Inventor: LEE, Jae Won, Seoul 156-786 (KR); PARK, Cheol Won, Seoul 120-762 (KR); JUN, Hun, Yongin-si Gyeonggi-do 448-525 (KR); LEE, Tae Won, Suwon-si Gyeonggi-do 443-811 (KR); KIM, Nam Hyuck, Seoul 137-793 (KR); LEE, Sun Young, Suwon-si Gyeonggi-do 442-816 (KR); YU, Ji Hyuck, Seoul 122-735 (KR)
(74) Representative: Schiweck Weinzierl Koch Patentanwälte Partnerschaft mbB
(86) International application number: PCT/KR2014/012677
(87) International publication number: WO 2015/102282

(56) References cited:
- EP-A1- 2 106 789
- EP-A1- 2 322 175
- WO-A1-2013/159166
- WO-A1-2014/035190
- WO-A2-2011/142621
- AU-A1- 2008 201 290
- KR-A- 20090 114 190
- KR-A- 20130 024 940
- US-A1- 2005 107 446
- US-A1- 2011 213 004
- US-A1- 2012 021 049
- US-A1- 2012 045 505
- DATABASE EMBASE [Online] ELSEVIER SCIENCE PUBLISHERS, AMSTERDAM, NL; 2011, HU X -L ET AL: "Effect of combination of antihypertensive and lipid lowering therapy on arterial stiffness in elderly patients with mild to moderate essential hypertension", XP002769803, Database accession no. EMB-2011547668 & CHINESE JOURNAL OF EVIDENCE-BASED MEDICINE 2011 WEST CHINA UNIVERSITY OF MEDICAL SCIENCE CHN, vol. 11, no. 9, 2011, pages 1008-1011, ISSN: 1672-2531

## Description

### TECHNICAL FIELD

The present invention relates to a pharmaceutical combination preparation comprising an angiotensin-II receptor blocker and an HMG-CoA reductase inhibitor and a pharmaceutically acceptable alkalinizing agent, wherein the angiotensin-II receptor blocker is candesartan cilexetil, the HMG-CoA reductase inhibitor is rosuvastatin, and the alkalinizing agent is calcium carbonate.

### BACKGROUND ART

Hypertension requiring drug therapy is defined as persistently showing diastolic blood pressure (DBP) of 90 mmHg or higher or systolic blood pressure (SBP) of 140 mmHg or higher. Hypertension is the result of increased tension in the peripheral vascular smooth muscle, and causes an increase in arteriolar resistance and a reduction in venous volume. Most hypertension patients have no symptoms, but chronic hypertension may cause congestive heart failure, myocardial infarction, renal impairment, cerebral hemorrhage, etc. in both hypertensive DBP and SBP. Blood pressure is regulated by sympathetic nervous system-mediated baroreceptor reflex and the renin-angiotensin-aldosterone system. Most antihypertensives lower blood pressure by reducing cardiac output or peripheral resistance. The renal blood flow is regulated by the renin-angiotensin-aldosterone system, which contributes to the long-term regulation of blood pressure. Baroreceptors in the kidney respond to reduced arterial pressure by releasing rennin, which converts angiotensinogen into angiotensin I, and angiotensin I is converted into angiotensin II by the action of an angiotensin converting enzyme. Angiotensin II is the most potent circulating vasoconstrictor to increase blood pressure, and stimulates aldosterone secretion, leading to increased renal sodium resorption and an increase in blood volume, which contribute to a further increase in blood pressure.

Angiotensin-II receptor blockers (ARBs) are used instead of angiotensin converting enzyme inhibitors (ACE inhibitor). Angiotensin-II receptor is a powerful circulatory vasoconstrictor, and an angiotensin-II receptor blocker includes losartan, candesartan, valsartan, telmisartan, irbesartan, olmesartan, etc., and candesartan and olmesartan are known to have the most excellent anti-hypertensive effects. These drugs have an antagonistic action against angiotensin II receptors to cause vasodilation and to block aldosterone secretion, leading to the decrease in blood pressure. Further, angiotensin-II receptor blockers have pharmacological actions that are similar to those of ACE inhibitors, but they have an advantage of remarkably reducing the risk of cough, angioedema, etc.

Dyslipidemia is a primary contributor to coronary heart disease (CHD), and is a disease that causes approximately half of all deaths occurring in the US. Dyslipidemia can be caused by a simple genetic defect in the lipid metabolism or a combination of genetic and life-style factors. By drug therapy and proper life-style changes for dyslipidemia, progression of coronary plaque, recurrence of previous lesion and mortality due to CHD can be reduced up to 30∼40 %. Occurrence of CHD is highly related to elevated cholesterol, and in particular, strongly related to a high level of Low Density Lipoprotein (LDL) cholesterol in blood. Therefore, Lowering LDL concentration is the primary target of cholesterol-lowering therapy. Among various therapies recommended on the basis of the risk level of CHD, HMG-CoA reductase inhibitor which is a cholesterol biosynthesis inhibitor is known as one of the most effective antihyperlipidemic drugs.

HMG-CoA reductase inhibitor inhibits the enzyme activity by effectively competing with HMG-CoA reductase, which is the rate-limiting enzyme in cholesterol synthesis in hepatocytes. Its efficacy leads to a depletion of intracellular cholesterol and a reduction of cholesterol required for synthesis of bile acid, which induces an upregulation of the number of LDL receptors, which in turn promotes a lowering of LDL levels in blood. The drugs used as HMG-CoA reductase inhibitors include lovastatin, simvastatin, pravastatin, atorvastatin, fluvastatin, rosuvastatin, etc. Of them, rosuvastatin and atorvastatin are known as the most powerful cholesterol-lowering statin drugs.

In particular, there are many cases in which hypertension patients with dyslipidemia are treated with a combination of ARBs and HMG-CoA reductase inhibitors in order to reduce the risk of cardiovascular diseases such as atheriosclerosis, coronary artery disease, etc. over the long term (Korean Patent No. 10-1129767). These combination therapies are pharmacologically advantageous in that they reduce the size of vascular plaque, decrease production of nitric oxide (NO) to inhibit platelet aggregation, prevent accumulation of lipids within the arterial wall, and reduce oxLDL and phospholipase activity, thereby inhibiting occurrence of dyslipidemia.

AU 2008 201 290 discloses a pharmaceutical composition comprising rosuvastatin and candesartan cilexetil.

WO 2011/142621 describes a pharmaceutical formulation in the form of bilayered tablets consisting of a first layer containing irebesartan or pharmaceutically acceptable salts thereof and a second layer containing an HMG-CoA reductase inhibitor and a basic additive.

WO 2014/035190 discloses a pharmaceutical composition capsule formulation comprising an independent irbesartan unit comprising irbesartan or a pharmaceutically acceptable salt thereof, and an independent HMG-CoA reductase inhibitor unit comprising an HMG-CoA reductase inhibitor or a pharmaceutically acceptable salt thereof, and an alkaline additive, wherein said independent units are separated from each other within a capsule, and a method for preparing the same.

WO 2013/159166 describes a pharmaceutical form comprising one or more HMG-CoA reductase inhibitors, one or more angiotensin II receptor antagonists and one or more thiazide class diuretics.

EP 2 106 789 relates to a solid pharmaceutical composition comprising candesartan or a salt, solvate or ester thereof, a polymeric agent comprising a copolymer of polyvinyl alcohol and polyethylene glycol, and optionally at least one other excipient as well as a process for preparing the composition.

US 2011/213004 discloses a combination therapy and a combination preparation of an angiotensin-II-receptor blocker and an HMG-CoA reductase inhibitor characterized in that the angiotensin-II-receptor blocker is absorbed substantially later than the HMG-CoA reductase inhibitor.

EP 2 322 175 relates to a pharmaceutical composition comprising an angiotensin II receptor antagonist or a pharmaceutically acceptable salt thereof, pioglitazone or a pharmaceutically acceptable salt thereof, and rosuvastatin or a pharmaceutically acceptable salt thereof.

Hu X.-L. et al. ("Effect of combination of antihypertensive and lipid lowering therapy on arterial stiffness in elderly patients with mild to moderate essential hypertension"; Database Accession No. EMB-2011547668 & Chinese Journal of evidence-based Medicine 2011 West China University of Medical Science CHN, Vol. 11, No. 9, 2011, pages 1008-1011) deals with the combination of antihypertensive and lipid lowering therapy on arterial stiffness in elderly patients with mild to moderate essential hypertension.

However, most HMG-CoA reductase inhibitors exhibit unstable physical and chemical properties under acidic and oxidation conditions. It is known that the stability of angiotensin-II receptor blockers such as candesartan is drastically reduced according to changes in the physical pressure and temperature by a tableting pressure and heat which are generated during a molding process of a pharmaceutical composition in a crystal form, in particular, a tablet form. Therefore, there is an urgent need to develop a combination preparation that is improved in stability and content uniformity of both drugs.

### DISCLOSURE

### Technical Problem

The present inventors have made extensive efforts to develop a stable, effective combination preparation. As a result, they have developed a pharmaceutical composition capable of improving stability and content uniformity of both an HMG-CoA reductase inhibitor and an angiotensin-II receptor blocker at the same time, and a preparation method thereof, thereby completing the present invention.

### Technical Solution

An object of the present invention is to provide a pharmaceutical combination preparation, comprising an angiotensin-II receptor blocker or a pharmaceutically acceptable salt thereof and an HMG-CoA reductase inhibitor or a pharmaceutically acceptable salt thereof, and a pharmaceutically acceptable alkalinizing agent, wherein the angiotensin-II receptor blocker is candesartan cilexetil, the HMG-CoA reductase inhibitor is rosuvastatin, and the alkalinizing agent is calcium carbonate. The inventive pharmaceutical combination preparation comprises the alkalinizing agent in an amount of 0.3 to 10% by weight, based on the total weight of the pharmaceutical combination preparation and/or the lubricant polyethylene glycol. The pharmaceutical combination preparation has effectively improved stability of both drugs by adding the alkalinizing agent, and furthermore in stability and processability of the combination preparation by granulating the angiotensin-II receptor blocker, separating the HMG-CoA reductase inhibitor, mixing them together, and then adding a proper lubricant thereto.

### Advantageous Effects

The pharmaceutical combination preparation according to the present invention comprises an alkalinizing agent that does not affect stability of an angiotensin-II receptor blocker, thereby improving stability of the two drugs, angiotensin-II receptor blocker and HMG-CoA reductase inhibitor.

Further, the angiotensin-II receptor blocker and the HMG-CoA reductase inhibitor are not granulated simultaneously, but separately prepared and mixed to minimize change in the stability of the angiotensin-II receptor blocker during molding of the tablet.

Further, the pharmaceutical combination preparation according to the present invention is prepared by selecting and using a proper lubricant, thereby improving stability of the both drugs and uniformity of formulation.

### DESCRIPTION OF DRAWINGS

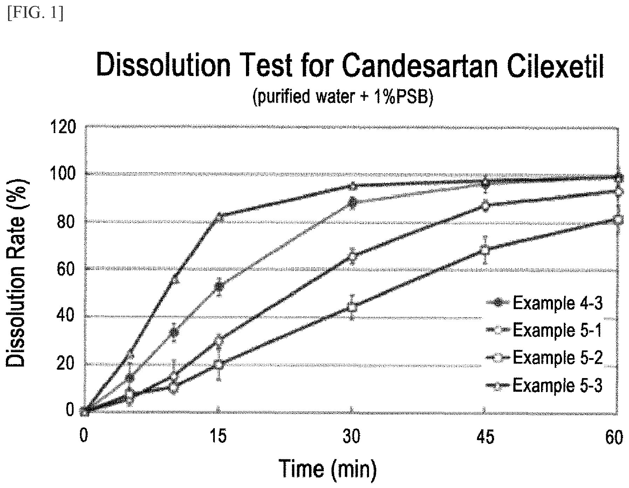

FIG. 1 is a graph showing the dissolution rate of candesartan cilexetil over time; and

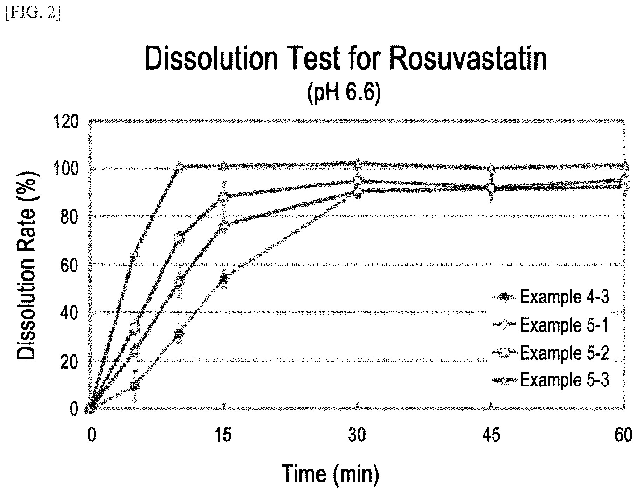

FIG. 2 is a graph showing the dissolution rate of rosuvastatin over time.

### BEST MODE

In order to achieve the above object, the present invention provides a pharmaceutical combination preparation, comprising an angiotensin-II receptor blocker or a pharmaceutically acceptable salt thereof; an HMG-CoA reductase inhibitor or a pharmaceutically acceptable salt thereof; and a pharmaceutically acceptable alkalinizing agent, wherein the angiotensin-II receptor blocker is candesartan cilexetil, the HMG-CoA reductase inhibitor is rosuvastatin, and the alkalinizing agent is calcium carbonate. The inventive pharmaceutical combination preparation comprises the alkalinizing in an amount of 0.3 to 10% by weight, based on the total weight of the pharmaceutical combination preparation and/or the lubricant polyethylene glycol.

The term 'angiotensin-II receptor blocker' refers to a drug having an antagonistic action against angiotensin-II receptor, which is responsible for strong vasoconstrictive action, and specific examples thereof may include losartan, candesartan, telmisartan, valsartan, irbesartan, olmesartan, etc., and pharmaceutically acceptable salts thereof. According to the present invention, the angiotensin-II receptor blocker is candesartan cilexetil. The angiotensin-II receptor blocker may be included in an amount of 5 to 1200 mg in the combination preparation of the present invention. Further, candesartan cilexetil may be preferably included in an amount of 8 to 32 mg in the combination preparation of the present invention.

As used herein, the term 'HMG-CoA' is an abbreviation of '3-hydroxy-methylglutaryl-coenzyme A', and means a precursor for the biosynthesis of sterols including cholesterol. As used herein, the term 'HMG-CoA reductase inhibitor' means a compound that provides an effect of lowering total cholesterol and LDL-cholesterol levels in the body by inhibiting activity of HMG-CoA reductase, which is involved in the conversion of HMG-CoA to mevalonate in the early stage of cholesterol biosynthesis. It is described herein that the HMG-CoA reductase inhibitor may be one or more selected from the group consisting of rosuvastatin, atorvastatin, pitavastatin, lovastatin, simvastatin, pravastatin, fluvastatin, and pharmaceutically acceptable salts thereof. The HMG-CoA reductase inhibitor according to the present invention is rosuvastatin. Preferably, the HMG-CoA reductase inhibitor may be included in an amount of 5 to 160 mg in the combination preparation of the present invention. Further, rosuvastatin may be preferably included in an amount of 5 to 40 mg in the combination preparation of the present invention.

As used herein, the term 'alkalinizing agent' is, also called 'alkalinizing agent' or 'alkalizing agent', a compound used to provide alkaline medium for product stability. Examples of alkalinizing agent may include calcium salts (calcium carbonate, calcium hydroxide, anhydrous calcium hydrogen phosphate, calcium dihydrogen phosphate, tricalcium phosphate, and hydrates thereof), magnesium salts (magnesium carbonate, magnesium hydroxide, magnesium silicate, magnesium oxide, magnesium aluminate, magnesium aluminum hydrate), lithium salts (lithium hydroxide), potassium salts (potassium hydroxide), sodium salts (sodium hydrogen carbonate, sodium borate, sodium carbonate, sodium hydroxide) or the like. According to the present invention, the alkalinizing agent is calcium carbonate. Also, it is possible to use a basic additive such as meglumine, arginine, etc., in addition to the stabilizer. The alkalinizing agent may be included in an amount of 0.001 to 40.0 % by weight, and preferably 0.3 to 10.0 % by weight, based on the total weight of the pharmaceutical combination preparation.

As used herein, the term 'pharmaceutically acceptable salt' means a form of the compound that does not cause significant irritation to an organism and does not abrogate the biological activity and properties of the administered angiotensin-II receptor blocker or HMG-CoA reductase inhibitor. The pharmaceutically acceptable salt may include acid addition salts formed by acids capable of forming a non-toxic acid addition salt containing pharmaceutically acceptable anions, for example, inorganic acids such as hydrochloric acid, sulfuric acid, nitric acid, phosphoric acid, hydrobromic acid, hydroiodic acid, etc.; organic carbonic acids such as tartaric acid, formic acid, citric acid, acetic acid, trichloroacetic acid, trifluoroacetic acid, gluconic acid, benzoic acid, lactic acid, fumaric acid, maleic acid, salicylic acid, etc.; or sulfonic acids such as methanesulfonic acid, ethanesulfonic acid, benzenesulfonic acid, p-toluenesulfonic acid, etc. Examples of pharmaceutically acceptable carboxylic acid salts include metal salts or alkaline earth metal salts such as lithium, sodium, potassium, calcium, magnesium, etc., amino acid salts such as lysine, arginine, guanidine, etc., organic salts such as dicyclohexylamine, N-methyl-D-glucamine, tris(hydroxymethyl)methylamine, diethanolamine, choline, triethylamine, etc.

Further, the combination preparation of the present invention may further comprise a pharmaceutically acceptable lubricant for the purpose of improving stability. As used herein, the term 'lubricant' collectively refers to a substance improving fluidity of a mixture during tableting. Specific example thereof may include talc, silicon dioxide, light anhydrous silicic acid, silica gel (Cab-O-Sil^{®}, Syloid^{®}, Aerosil^{®}), starch, calcium silicate, or polyethylene glycol (macrogol), etc., and preferably polyethylene glycol (macrogol) 6000. The lubricant may be included in an amount of 0.001 to 20 % by weight, and preferably, 0.3 to 5.0 % by weight, based on the total weight of the pharmaceutical combination preparation.

Further, the pharmaceutical combination preparation according to the present invention may further comprise a film layer on the external surface thereof. The film layer may be, for example, a light-shielding film layer, a moisture-proof film layer or a sugar film layer. The external film layer is preferably formed by a water-soluble material, and the water-soluble film-forming material may be hydroxypropylmethylcellulose (HPMC), hydroxypropylcellulose (HPC), hydroxyethylcellulose (HEC), celluloseacetatephthalate (CAP), ethylcellulose (EC), methylcellulose (MC), polymethacrylate, a polyvinylalcohol-polyethylene glycol graft copolymer (Kollicoat; registered trademark of BASF, German), polyvinyl alcohol (PVA) (Opadry; registered trademark of Colorcon, USA), and mixtures thereof.

Further, the pharmaceutical combination preparation of the present invention may be formulated by additionally using an additive typically used, such as a pharmaceutically acceptable diluent, binder, disintegrant, lubricant, pH adjuster, anti-foaming agent, solubilization aid, within the scope that does not impair the effect of the pharmaceutical combination preparation according to the present invention.

The pharmaceutical combination preparation of the present invention may be formulated into various forms, for example, a tablet such as a plain tablet, a film-coated tablet, a mono-layered tablet, a double-layered tablet, a multi-layered tablet or a cored tablet, a powder, a granule or a capsule.

In Examples and Experimental Examples of the present invention, among the combination preparations comprising a mixture of candesartan as an angiotensin-II receptor blocker and rosuvastatin as an HMG-CoA reductase inhibitor, a combination preparation prepared by mixing granulated candesartan with rosuvastatin using a binding liquid, that is, prepared by mixing the two drugs which are separately prepared (candesartan related compound 1.41 %, rosuvastatin related compound 0.05 %) showed remarkably excellent stability, compared to a combination preparation prepared by granulating the two drugs simultaneously (candesartan related compound 2.50 %, rosuvastatin related compound 1.31 %) (Example 3). Furthermore, when polyethylene glycol was added as a lubricant, the formulation had more excellent stability (Experimental Example 4).

In order to achieve the above object, the present invention provides a method for preparing the pharmaceutical combination preparation, comprising: (a) preparing granules by mixing an angiotensin-II receptor blocker or a pharmaceutically acceptable salt thereof with a binding liquid; (b) mixing the granules with an HMG-CoA reductase inhibitor or a pharmaceutically acceptable salt thereof; and (c) preparing a tablet comprising the mixture of step (b).

Step (a) is a step of preparing granules comprising the angiotensin-II receptor blocker or the pharmaceutically acceptable salt thereof.

In the present invention, the granules may be prepared by mixing the angiotensin-II receptor blocker or the pharmaceutically acceptable salt thereof with the binding liquid.

As used herein, the term 'binding liquid' is, also called 'binder', a substance that imparts cohesiveness to the pharmaceutical formulation, in which cohesiveness ensures that the composition remains intact within the formulation especially in case of tablets after compression. Depending on the compaction technique used (direct compression, dry granulation or wet granulation), different binders may be used. i) Suitable binders for dry compaction techniques (direct compression and dry granulation) are exemplified by lactose, sucrose, mannitol, sorbitol, saccharose, starch, pregelatinized starch, microcrystalline cellulose, powdered cellulose, calcium hydrogen phosphate, calcium carbonate and any combinations thereof. ii) In wet granulation processes, binders can be used as a solution, and suitable binders may be exemplified by polyvinylpyrrolidone, dispersible cellulose, hydroxypropyl cellulose, hydroxypropylmethyl cellulose, methylcellulose, starch, pregelatinized starch, partly pregelatinized starch, gum arabic, dextrin, pullulan, etc. Preferably, as in Examples of the present invention, hydroxypropyl cellulose which is dissolved in ethanol, etc. and then dried may be used.

Step (b) is a step of mixing the granules with the HMG-CoA reductase inhibitor or the pharmaceutically acceptable salt thereof.

The angiotensin-II receptor blocker and the HMG-CoA reductase inhibitor are not granulated simultaneously, but mixed them prepared separately, thereby minimizing a change in the stability of the angiotensin-II receptor blocker upon molding of the tablet. In Step (b), therefore, the granules containing the angiotensin-II receptor blocker is mixed with the HMG-CoA reductase inhibitor to prepare a mixture. Preferably, the mixture may further include a lubricant such as polyethylene glycol, etc.

Step (c) is a step of preparing a tablet comprising the mixture of step (b). The process associated in the preparation of the pharmaceutical combination preparation according to the present invention may be performed in accordance with the typical process of the pharmaceutical field. A specific example thereof may be a compression molding method, as in the Examples of the present invention.

### MODE FOR INVENTION

Hereinafter, the present invention will be described in more detail with reference to the Examples.

### Example 1: Preparation of candesartan cilexetil granules

### Example 1-1

Step 1: 3.2 g of candesartan cilexetil, 12.5 g of microcrystalline cellulose, 12.8 g of lactose, 0.5 g of calcium carboxymethylcellulose, and 0.7 g of hydroxypropylcellulose were mixed.
Step 2: The mixture of Step 1 was passed through an 18 mesh sieve, and then mixed with 0.3 g of magnesium stearate to prepare a mixture.

### Example 1-2

Step 1: 0.7 g of hydroxypropylcellulose was dissolved in 5.0 g of ethanol to prepare a binding liquid.
Step 2: 3.2 g of candesartan cilexetil, 12.5 g of microcrystalline cellulose, 12.8 g of lactose, and 0.5 g of calcium carboxymethylcellulose were mixed.
Step 3: The binding liquid of Step 1 was added to and mixed with the mixture of Step 2. The resulting mixture was dried at 50 °C for 2 hours, and passed through an 18 mesh sieve.
Step 4: The granules of Step 3 was mixed with 0.3 g of magnesium stearate to prepare granules.

### Example 1-3

A candesartan cilexetil tablet was prepared in the same manner as in Example 1-2, except that purified water was used instead of ethanol as the binding liquid in Step 1 of Example 1-2.

### Example 1-4

Step 1: 3.2 g of candesartan cilexetil, 12.5 g of microcrystalline cellulose, 12.8 g of lactose, 0.5 g of calcium carboxymethylcellulose, and 0.7 g of hydroxypropylcellulose were mixed.
Step 2: The mixture of Step 1 was compression-molded, and then passed through an 18 mesh sieve.
Step 3: The granules of Step 3 was mixed with 0.3 g of magnesium stearate to prepare granules.

### Experimental Example 1: Test of physical properties of candesartan cilexetil granules

Content uniformity of the candesartan cilexetil granules prepared in Examples 1-1 to 1-4 was measured and the results are shown in the following Table 1.

**[Table 1]**

| | Content uniformity of candesartan cilexetil | | | | | | | |
|---|---|---|---|---|---|---|---|---|
| | Content 1 | Content 2 | Content 3 | Content 4 | Content 5 | Content 6 | Mean | Standard deviation |
| Example 1-1 | 111.2% | 89.7% | 93.3% | 87.3% | 109.5% | 83.2% | 95.7% | 0.118 |
| Example 1-2 | 100.4% | 101.2% | 99.2% | 100.6% | 100.9% | 100.8% | 100.8% | 0.007 |
| Example 1-3 | 100.8% | 97.8% | 101.2% | 100.3% | 100.5% | 97.3% | 99.7% | 0.017 |
| Example 1-4 | 95.0% | 94.5% | 93.0% | 94.0% | 96.5% | 98.9% | 95.3% | 0.021 |

Further, the candesartan cilexetil granules prepared in Examples 1-1 to 1-4 were stored at 60 °C for 1 week, and then changes in the candesartan cilexetil content and total related compound over time were measured and the results are shown in the following Table 2.

**[Table 2]**

| | Candesartan cilexetil content | | Total related compound | |
|---|---|---|---|---|
| | Day 0 | Day 7 | Day 0 | Day 7 |
| Example 1-1 | 95.7% | 98.3% | 0.27% | 0.43% |
| Example 1-2 | 100.8% | 99.2% | 0.26% | 0.67% |
| Example 1-3 | 99.7% | 97.9% | 0.27% | 0.71% |
| Example 1-4 | 95.3% | 92.3% | 0.47% | 2.01% |

As shown in Table 1, the mixture had poor content uniformity when no binding liquid was used, whereas the granules had improved content uniformity when granules were prepared by using the binding liquid or by compression-molding.

As shown in Table 2, when granules were prepared by compression-molding, the stability of the main ingredient candesartan cilexetil was low, compared to those of other granules. The granules of Example 1-1 had the most excellent stability, and the granules of Example 1-2 prepared by using ethanol as the binding liquid was maintained stably.

### Example 2: Preparation of candesartan cilexetil tablet by use of alkalinizing agent

### Example 2-1

Step 1: 0.7 g of hydroxypropylcellulose was dissolved in 5.0 g of ethanol to prepare a binding liquid.
Step 2: 3.2 g of candesartan cilexetil, 12.5 g of microcrystalline cellulose, 12.8 g of lactose, and 0.5 g of calcium carboxymethylcellulose were mixed.
Step 3: The mixture of Step 2 was mixed with the binding liquid of step 1, dried at 50 °C for 2 hours, and then sieved to prepare granules.
Step 4: The granules of Step 3 was mixed with 0.3 g of magnesium stearate.
Step 5: The mixture of Step 4 was compression-molded to prepare a tablet, and at this time, the weight of one tablet was 150.0 mg.

### Example 2-2

A candesartan cilexetil tablet was prepared in the same manner as in Example 2-1, except that 11.8 g of lactose was used, and 1.0 g of sodium hydrogen carbonate was further used as an alkalinizing agent in Step 1 of Example 2-1.

### Example 2-3

A candesartan cilexetil tablet was prepared in the same manner as in Example 2-2, except that calcium carbonate was used instead of sodium hydrogen carbonate as an alkalinizing agent in Step 1 of Example 2-2.

### Example 2-4

A candesartan cilexetil tablet was prepared in the same manner as in Example 2-2, except that magnesium carbonate was used instead of sodium hydrogen carbonate as an alkalinizing agent in Step 1 of Example 2-2.

### Example 2-5

A candesartan cilexetil tablet was prepared in the same manner as in Example 2-2, except that calcium hydrogen phosphate dihydrate was used instead of sodium hydrogen carbonate as an alkalinizing agent in Step 1 of Example 2-2.

### Experimental Example 2: Test of stability of candesartan cilexetil tablet according to types of alkalinizing agent

The candesartan cilexetil tablets prepared in Examples 2-1 to 2-5 were stored at 60 °C for 1 week, and changes in the candesartan cilexetil content and total related compound over time were measured and the results are shown in the following Table 3.

**[Table 3]**

| | Candesartan cilexetil content | | Total related compound | |
|---|---|---|---|---|
| | Day 0 | Day 7 | Day 0 | Day 7 |
| Example 2-1 | 101.3% | 99.1% | 0.32% | 2.13% |
| Example 2-2 | 100.9% | 97.9% | 0.33% | 2.62% |
| Example 2-3 | 100.1% | 99.4% | 0.32% | 1.51% |
| Example 2-4 | 99.8% | 97.8% | 0.33% | 2.14% |
| Example 2-5 | 100.7% | 95.4% | 0.33% | 3.28% |

As shown in Table 3, stability of the main ingredient (candesartan cilexetil) was generally reduced by addition of the alkalinizing agent, but stability was improved in the tablet prepared by using calcium carbonate, compared to the tablet prepared without alkalinizing agent.

### Example 3: Preparation of combination preparation of candesartan cilexetil and rosuvastatin according to mixing mode

### Example 3-1

Step 1: 0.7 g of hydroxypropylcellulose was dissolved in 5.0 g of ethanol to prepare a binding liquid.
Step 2: 3.2 g of candesartan cilexetil, 2.08 g of rosuvastatin calcium, 12.5 g of microcrystalline cellulose, 12.8 g of lactose, and 0.4 g of calcium carboxymethylcellulose were mixed.
Step 3: The mixture of Step 2 was mixed with the binding liquid of Step 1, dried at 50 °C for 2 hours, and then sieved to prepare granules.
Step 4: The granules of Step 3 was mixed with 0.32 g of magnesium stearate.
Step 5: The mixture of step 4 was compression-molded to prepare a tablet, and at this time, the weight of one tablet was 160.0 mg.

### Example 3-2

A candesartan cilexetil tablet was prepared in the same manner as in Example 3-1, except that 11.8 g of lactose was used and 1.0 g of calcium carbonate was further used as an alkalinizing agent in Step 1 of Example 3-1.

### Example 3-3

Step 1: 0.7 g of hydroxypropylcellulose was dissolved in 5.0 g of ethanol to prepare a binding liquid.
Step 2: 3.2 g of candesartan cilexetil, 12.5 g of microcrystalline cellulose, 12.8 g of lactose, and 0.4 g of calcium carboxymethylcellulose were mixed.
Step 3: The mixture of Step 2 was mixed with the binding liquid of Step 1, and dried at 50 °C for 2 hours to prepare granules.
Step 4: The granules of step 3 was mixed with 2.08 g of rosuvastatin calcium and 0.32 g of magnesium stearate.
Step 5: The mixture of step 4 was compression-molded to prepare a tablet, and at this time, the weight of one tablet was 160.0 mg.

### Example 3-4

A candesartan cilexetil tablet was prepared in the same manner as in Example 3-3, except that 11.8 g of lactose was used and 1.0 g of calcium carbonate was further used as an alkalinizing agent in Step 1 of Example 3-3.

### Experimental Example 3: Test of stability of combination preparation of candesartan cilexetil and rosuvastatin according to mixing mode

The combination preparations of candesartan cilexetil and rosuvastatin prepared in Examples 3-1 to 3-4 were stored at 60 °C for 1 week, and changes in two main ingredients over time were measured. The results are shown in the following Tables 4 and 5.

**[Table 4]**

| | Candesartan cilexetil content | | Total related compound | |
|---|---|---|---|---|
| | Day 0 | Day 7 | Day 0 | Day 7 |
| Example 3-1 | 99.2% | 97.4% | 0.47% | 2.59% |
| Example 3-2 | 100.9% | 97.8% | 0.49% | 2.50% |
| Example 3-3 | 100.1% | 98.8% | 0.32% | 1.86% |
| Example 3-4 | 99.9% | 99.2% | 0.32% | 1.64% |

**[Table 5]**

| | Rosuvastatin content | | Total related compound | |
|---|---|---|---|---|
| | Day 0 | Day 7 | Day 0 | Day 7 |
| Example 3-1 | 98.7% | 97.6% | 0.03% | 1.61% |
| Example 3-2 | 99.2% | 98.7% | 0.03% | 1.31% |
| Example 3-3 | 98.9% | 99.3% | 0.03% | 0.20% |
| Example 3-4 | 99.5% | 99.8% | 0.03% | 0.12% |

As shown in Tables 4 and 5, upon preparation of the combination preparations of candesartan cilexetil and rosuvastatin, Experimental Example prepared by mixing the two main ingredients simultaneously using the binding liquid showed low stability in both main ingredients, whereas Experimental Example prepared by separately preparing and mixing them showed stability improvement in both main ingredients. Further, Experimental Example prepared by using calcium carbonate as an alkalinizing agent showed stability improvement in both main ingredients.

### Example 4: Preparation of combination preparation of candesartan cilexetil and rosuvastatin by types of lubricant

### Example 4-1

Step 1: 0.7 g of hydroxypropylcellulose was dissolved in 5.0 g of ethanol to prepare a binding liquid.
Step 2: 3.2 g of candesartan cilexetil, 12.5 g of microcrystalline cellulose, 11.0 g of lactose, 0.4 g of calcium carboxymethylcellulose, and 1.0 g of calcium carbonate were mixed.
Step 3: The mixture of Step 2 was mixed with the binding liquid of Step 1, dried at 50 °C for 2 hours, and then sieved to prepare granules.
Step 4: The granules of Step 3 was mixed with 2.08 g of rosuvastatin calcium, 0.8 g of talc, and 0.32 g of magnesium stearate.
Step 5: The mixture of step 4 was compression-molded to prepare a tablet, and at this time, the weight of one tablet was 160.0 mg.

### Example 4-2

A candesartan cilexetil tablet was prepared in the same manner as in Example 4-1, except that light anhydrous silicic acid was used instead of talc as a lubricant in Step 4 of Example 4-1.

### Example 4-3

A candesartan cilexetil tablet was prepared in the same manner as in Example 4-1, except that macrogol (polyethylene glycol) 6000 was used instead of talc as a lubricant in Step 4 of Example 4-1.

### Experimental Example 4: Test of formulation uniformity of combination preparation of candesartan cilexetil and rosuvastatin by use of lubricant

Content uniformity of the combination preparations of candesartan cilexetil and rosuvastatin prepared in Example 3-4 and Examples 4-1 to 4-3 was measured, and the results are shown in the following Table 6.

**[Table 6]**

| | Content uniformity of rosuvastatin | | | | | | | |
|---|---|---|---|---|---|---|---|---|
| | Content 1 | Content 2 | Content 3 | Content 4 | Content 5 | Content 6 | Mean | Standard deviation |
| Example 3-4 | 123.7% | 100.3% | 84.2% | 77.5% | 110.8% | 100.2% | 99.5% | 0.169 |
| Example 4-1 | 95.7% | 88.2% | 104.4% | 101.0% | 110.3% | 96.4% | 99.3% | 0.077 |
| Example 4-2 | 102.6% | 100.4% | 101.0% | 102.0% | 102.1% | 100.2% | 101.4% | 0.010 |
| Example 4-3 | 96.3% | 103.1% | 98.2% | 95.0% | 95.0% | 103.4% | 99.1% | 0.034 |

Further, the combination preparations of candesartan cilexetil and rosuvastatin prepared in Example 3-4 and Examples 4-1 to 4-3 were stored at 60 °C for 1 week, and changes in the content of two main ingredients over time and total related compound were measured. The results are shown in the following Tables 7 and 8.

**[Table 7]**

| | Candesartan cilexetil content | | Total related compound | |
|---|---|---|---|---|
| | Day 0 | Day 7 | Day 0 | Day 7 |
| Example 3-4 | 99.9% | 99.2% | 0.32% | 1.64% |
| Example 4-1 | 99.8% | 97.2% | 0.33% | 1.99% |
| Example 4-2 | 100.7% | 96.3% | 0.37% | 2.94% |
| Example 4-3 | 100.4% | 99.5% | 0.32% | 0.70% |

**[Table 8]**

| | Candesartan cilexetil content | | Total related compound | |
|---|---|---|---|---|
| | Day 0 | Day 7 | Day 0 | Day 7 |
| Example 3-4 | 99.5% | 99.8% | 0.03% | 0.12% |
| Example 4-1 | 99.3% | 98.7% | 0.04% | 0.57% |
| Example 4-2 | 101.4% | 97.2% | 0.04% | 1.28% |
| Example 4-3 | 99.1% | 99.9% | 0.03% | 0.09% |

As shown in Table 6, upon preparation of combination preparations of candesartan cilexetil and rosuvastatin, when no lubricant was used during mixing with rosuvastatin, the formulation uniformity of the main ingredient (rosuvastatin) was reduced. In contrast, when the lubricant was used, the formulation uniformity was improved.

Further, as shown in Tables 7 and 8, stability of the two main ingredients was greatly reduced by using light anhydrous silicic acid as the lubricant, whereas stability of the two main ingredients was improved by using polyethylene glycol 6000.

### Example 5: Preparation of combination preparation of candesartan cilexetil and rosuvastatin by use of disintegrant

### Example 5-1

Step 1: 0.7 g of hydroxypropylcellulose was dissolved in 5.0 g of ethanol to prepare a binding liquid.
Step 2: 3.2 g of candesartan cilexetil, 12.5 g of microcrystalline cellulose, 11.0 g of lactose, 0.2 g of calcium carboxymethylcellulose, and 1.0 g of calcium carbonate were mixed.
Step 3: The mixture of Step 2 was mixed with the binding liquid of step 1, dried at 50 °C for 2 hours, and then sieved to prepare granules.
Step 4: The granules of step 3 was mixed with 2.08 g of rosuvastatin calcium, 0.2 g of calcium carboxymethylcellulose, 0.8 g of polyethylene glycol 6000, and 0.32 g of magnesium stearate.
Step 5: The mixture of Step 4 was compression-molded to prepare a tablet, and at this time, the weight of one tablet was 160.0 mg.

### Example 5-2

A candesartan cilexetil tablet was prepared in the same manner as in Example 5-1, except that no calcium carboxymethylcellulose was used as a disintegrant in Step 2 of Example 5-1, and 0.4 g of calcium carboxymethylcellulose was used as a disintegrant in Step 4.

### Example 5-3

A candesartan cilexetil tablet was prepared in the same manner as in Example 5-1, except that 11.7 g of microcrystalline cellulose was used in Step 2 of Example 5-1, and 1.0 g of calcium carboxymethylcellulose was used as a disintegrant in Step 4.

### Experimental Example 5: Test of dissolution of combination preparation of candesartan cilexetil and rosuvastatin by use of disintegrant

The combination preparations of candesartan cilexetil and rosuvastatin prepared in Example 4-3 and Examples 5-1 to 5-3 were subjected to a dissolution test under the conditions of water + 1 % PSB, pH 6.6, 900 ml, and 50 rpm by a paddle method, and the results are shown in the following Table 9 and 10.

**[Table 9]**

| | Dissolution test for candesartan cilexetil (water + 1 % PSB) | | | | | |
|---|---|---|---|---|---|---|
| | 5 min | 10 min | 15 min | 30 min | 45 min | 60 min |
| Example 4-3 | 14.45% | 33.4% | 52.8% | 88.4% | 96.5% | 99.6% |
| Example 5-1 | 5.5% | 15.2% | 30.0% | 65.7% | 87.4% | 93.6% |
| Example 5-2 | 7.5% | 10.3% | 20.1% | 44.4% | 68.7% | 81.9% |
| Example 5-3 | 24.6% | 56.1% | 82.5% | 95.6% | 97.8% | 99.3% |

**[Table 10]**

| | Dissolution test for rosuvastatin (pH 6.6) | | | | | |
|---|---|---|---|---|---|---|
| | 5 min | 10 min | 15 min | 30 min | 45 min | 60 min |
| Example 4-3 | 95% | 31.4% | 54.4% | 90.4% | 92.1% | 92.7% |
| Example 5-1 | 24.0% | 53.0% | 76.4% | 90.8% | 91.7% | 92.4% |
| Example 5-2 | 33.9% | 71.2% | 88.3% | 95.1% | 92.2% | 95.3% |
| Example 5-3 | 65.0% | 101.1% | 101.3% | 102.2% | 100.6% | 101.7% |

As shown in Tables 9 and 10, the dissolution rate of the main ingredient (candesartan cilexetil) was influenced by the amount of the disintegrant which was mixed during the preparation of candesartan cilexetil granules, and the dissolution rate of the main ingredient (rosuvastatin) was influenced by the amount of the disintegrant which was mixed upon mixing of rosuvastatin, indicating that independent control of dissolution profiles of the two main ingredients is possible.

## Claims

1. A pharmaceutical combination preparation, comprising:
(a) an angiotensin-II receptor blocker or a pharmaceutically acceptable salt thereof, an HMG-CoA reductase inhibitor or a pharmaceutically acceptable salt thereof, and a pharmaceutically acceptable alkalinizing agent,
wherein the angiotensin-II receptor blocker is candesartan cilexetil, the HMG-CoA reductase inhibitor is rosuvastatin, and the alkalinizing agent is calcium carbonate, and wherein the alkalinizing agent is included in an amount of 0.3 to 10% by weight, based on the total weight of the pharmaceutical combination preparation; and/or
(b) an angiotensin-II receptor blocker or a pharmaceutically acceptable salt thereof, an HMG-CoA reductase inhibitor or a pharmaceutically acceptable salt thereof,
a pharmaceutically acceptable alkalinizing agent, and
a lubricant,
wherein the angiotensin-II receptor blocker is candesartan cilexetil, the HMG-CoA reductase inhibitor is rosuvastatin, the alkalinizing agent is calcium carbonate, and the lubricant is polyethylene glycol.

2. The pharmaceutical combination preparation according to claim 1, wherein the pharmaceutical combination is formulated as tablet; preferably a plain tablet, a film-coated tablet, a mono-layered tablet, a double-layered tablet, a multi-layered tablet or a cored tablet; a powder, a granule or a capsule.

3. The pharmaceutical combination preparation according to claim 1, wherein the lubricant is included in an amount of 0.3 to 5.0% by weight, based on the total weight of the pharmaceutical combination preparation.

4. A method for preparing the pharmaceutical combination preparation , comprising:
(a) preparing granules by mixing an angiotensin-II receptor blocker or a pharmaceutically acceptable salt thereof with a binding liquid;
(b) mixing the granules with an HMG-CoA reductase inhibitor or a pharmaceutically acceptable salt thereof; and
(c) preparing a tablet comprising the mixture of step (b),
wherein the pharmaceutical combination preparation comprises an angiotensin-II receptor blocker or a pharmaceutically acceptable salt thereof, an HMG-CoA reductase inhibitor or a pharmaceutically acceptable salt thereof, and a pharmaceutically acceptable alkalinizing agent, and wherein the angiotensin-II receptor blocker is candesartan cilexetil, the HMG-CoA reductase inhibitor is rosuvastatin, and the alkalinizing agent is calcium carbonate.

5. The method according to claim 4, wherein the pharmaceutical combination preparation is formulated as tablet; preferably a plain tablet, a film-coated tablet, a mono-layered tablet, a double-layered tablet, a multi-layered tablet or a cored tablet; a powder, a granule or a capsule.

6. The method according to claim 4, wherein the alkalinizing agent is included in an amount of 0.3 to 10% by weight, based on the total weight of the pharmaceutical combination preparation.

7. The method according to claim 4, wherein the pharmaceutical combination preparation further comprises a lubricant.

8. The method according to claim 7, wherein the lubricant is included in an amount of 0.3 to 5.0% by weight, based on the total weight of the pharmaceutical combination preparation.

9. The method according to claim 7, wherein the lubricant is polyethylene glycol.

10. The method according to claim 4, wherein the mixture of Step (b) further comprises a lubricant.

## Patentansprüche

1. Pharmazeutisches Kombinationspräparat, umfassend:
(a) einen Angiotensin-II-Rezeptor-Blocker oder ein pharmazeutisch akzeptables Salz davon, einen HMG-CoA-Reduktase-Inhibitor oder ein pharmazeutisch akzeptables Salz davon, und ein pharmazeutisch akzeptables Alkalisierungsmittel,
wobei der Angiotensin-II-Rezeptor-Blocker Candesartancilexetil ist, der HMG-CoA-Reduktase-Inhibitor Rosuvastatin ist und das Alkalisierungsmittel Calciumcarbonat ist, und wobei das Alkalisierungsmittel in einer Menge von 0,3 bis 10 Gew.-%, basierend auf dem Gesamtgewicht des pharmazeutischen Kombinationspräparats, enthalten ist, und/oder
(b) einen Angiotensin-II-Rezeptor-Blocker oder ein pharmazeutisch akzeptables Salz davon, einen HMG-CoA-Reduktase-Inhibitor oder ein pharmazeutisch akzeptables Salz davon,
ein pharmazeutisch akzeptables Alkalisierungsmittel, und
ein Schmiermittel,
wobei der Angiotensin-II-Rezeptor-Blocker Candesartancilexetil ist, der HMG-CoA-Reduktase-Inhibitor Rosuvastatin ist, das Alkalisierungsmittel Calciumcarbonat ist und das Schmiermittel Polyethylenglykol ist.

2. Pharmazeutisches Kombinationspräparat nach Anspruch 1, wobei die pharmazeutische Kombination als Tablette formuliert ist; vorzugsweise eine einfache Tablette, eine Filmtablette, eine einschichtige Tablette, eine doppelschichtige Tablette, eine mehrschichtige Tablette oder eine Tablette mit Kern; ein Pulver, ein Granulat oder eine Kapsel.

3. Pharmazeutisches Kombinationspräparat nach Anspruch 1, wobei das Schmiermittel in einer Menge von 0,3 bis 5,0 Gew.-%, basierend auf dem Gesamtgewicht des pharmazeutischen Kombinationspräparats, enthalten ist.

4. Verfahren zur Herstellung des pharmazeutischen Kombinationspräparats, umfassend:
(a) Herstellen von Granulat durch Mischen eines Angiotensin-II-Rezeptor-Blockers oder eines pharmazeutisch akzeptablen Salzes davon mit einer Bindeflüssigkeit;
(b) Mischen des Granulats mit einem HMG-CoA-Reduktase-Inhibitor oder einem pharmazeutisch akzeptablen Salz davon; und
(c) Herstellen einer Tablette, umfassend die Mischung von Schritt (b),
wobei das pharmazeutische Kombinationspräparat einen Angiotensin-II-Rezeptor-Blocker oder ein pharmazeutisch akzeptables Salz davon, einen HMG-CoA-Reduktase-Inhibitor oder ein pharmazeutisch akzeptables Salz davon und ein pharmazeutisch akzeptables Alkalisierungsmittel umfasst, und wobei der Angiotensin-II-Rezeptor-Blocker Candesartancilexetil ist, der HMG-CoA-Reduktase-Inhibitor Rosuvastatin ist und das Alkalisierungsmittel Calciumcarbonat ist.

5. Verfahren nach Anspruch 4, wobei das pharmazeutische Kombinationspräparat als Tablette formuliert ist; vorzugsweise eine einfache Tablette, eine Filmtablette, eine einschichtige Tablette, eine doppelschichtige Tablette, eine mehrschichtige Tablette oder eine Tablette mit Kern; ein Pulver, ein Granulat oder eine Kapsel.

6. Verfahren nach Anspruch 4, wobei das Alkalisierungsmittel in einer Menge von 0,3 bis 10 Gew.-%, basierend auf dem Gesamtgewicht des pharmazeutischen Kombinationspräparats, enthalten ist.

7. Verfahren nach Anspruch 4, wobei das pharmazeutische Kombinationspräparat ferner ein Schmiermittel umfasst.

8. Verfahren nach Anspruch 7, wobei das Schmiermittel in einer Menge von 0,3 bis 5,0 Gew.-%, basierend auf dem Gesamtgewicht des pharmazeutischen Kombinationspräparats, enthalten ist.

9. Verfahren nach Anspruch 7, wobei das Schmiermittel Polyethylenglykol ist.

10. Verfahren nach Anspruch 4, wobei die Mischung von Schritt (b) ferner ein Schmiermittel umfasst.

## Revendications

1. Préparation pharmaceutique combinée, comprenant :
(a) un bloqueur des récepteurs de l'angiotensine-II ou un sel pharmaceutiquement acceptable de celui-ci,
un inhibiteur de la HMG-CoA réductase ou un sel pharmaceutiquement acceptable de celui-ci, et
un agent alcalinisant pharmaceutiquement acceptable,
dans laquelle le bloqueur des récepteurs de l'angiotensine-II est le candésartan cilexétil, l'inhibiteur de la HMG-CoA réductase est la rosuvastatine, et l'agent alcalinisant est le carbonate de calcium, et dans laquelle l'agent alcalinisant est inclus en une quantité de 0,3 à 10 % en poids, sur la base du poids total de la préparation de combinaison pharmaceutique ; et/ou
(b) un bloqueur des récepteurs de l'angiotensine-II ou un sel pharmaceutiquement acceptable de celui-ci,
un inhibiteur de la HMG-CoA réductase ou un sel pharmaceutiquement acceptable de celui-ci,
un agent alcalinisant pharmaceutiquement acceptable, et
un lubrifiant,
dans laquelle le bloqueur des récepteurs de l'angiotensine-II est le candésartan cilexétil, l'inhibiteur de la HMG-CoA réductase est la rosuvastatine, l'agent alcalinisant est le carbonate de calcium, et le lubrifiant est le polyéthylèneglycol.

2. Préparation de combinaison pharmaceutique selon la revendication 1, dans laquelle la combinaison pharmaceutique est formulée sous forme de comprimé; de préférence un comprimé ordinaire, un comprimé pelliculé, un comprimé mono-couche, un comprimé double-couche, un comprimé multicouche ou un comprimé à noyau; une poudre, un granulé ou une capsule.

3. Préparation de combinaison pharmaceutique selon la revendication 1, dans laquelle le lubrifiant est inclus en une quantité de 0,3 à 5,0% en poids, sur la base du poids total de la préparation de combinaison pharmaceutique.

4. Procédé pour la preparation d'une combinaison pharmaceutique, comprenant les étapes suivantes:
(a) préparer des granules en mélangeant un bloqueur des récepteurs de l'angiotensine II ou un sel pharmaceutiquement acceptable de celui-ci avec un liquide de liaison;
(b) mélanger les granules avec un inhibiteur de la HMG-CoA réductase ou un sel pharmaceutiquement acceptable de celui-ci; et
(c) préparer un comprimé comprenant le mélange de l'étape (b),
dans laquelle la préparation d'association pharmaceutique comprend un bloqueur des récepteurs de l'angiotensine-II ou un sel pharmaceutiquement acceptable de celui-ci, un inhibiteur de la HMG-CoA réductase ou un sel pharmaceutiquement acceptable de celui-ci, et un agent alcalinisant pharmaceutiquement acceptable, et dans laquelle le bloqueur des récepteurs de l'angiotensine-II est le candésartan cilexétil, l'inhibiteur de la HMG-CoA réductase est la rosuvastatine, et l'agent alcalinisant est le carbonate de calcium.

5. Procédé selon la revendication 4, dans lequel la préparation de combinaison pharmaceutique est formulée sous forme de comprimé; de préférence un comprimé ordinaire, un comprimé pelliculé, un comprimé mono-couche, un comprimé double-couche, un comprimé multicouche ou un comprimé à noyau ; une poudre, un granulé ou une capsule.

6. Procédé selon la revendication 4, dans lequel l'agent alcalinisant est inclus en une quantité de 0,3 à 10 % en poids, sur la base du poids total de la préparation de combinaison pharmaceutique.

7. Procédé selon la revendication 4, dans lequel la préparation de combinaison pharmaceutique comprend en outre un lubrifiant.

8. Procédé selon la revendication 7, dans lequel le lubrifiant est inclus en une quantité de 0,3 à 5,0% en poids, sur la base du poids total de la préparation de combinaison pharmaceutique.

9. procédé selon la revendication 7, dans lequel le lubrifiant est du polyéthylène glycol.

10. Procédé selon la revendication 4, dans lequel le mélange de l'étape (b) comprend en outre un lubrifiant.
